(19) Europäisches Patentamt / European Patent Office / Office européen des brevets

(11) **EP 3 914 216 B1**

(12) **EUROPEAN PATENT SPECIFICATION**

(45) Date of publication and mention
of the grant of the patent:
**21.02.2024  Bulletin 2024/08**

(21) Application number: **20743954.8**

(22) Date of filing: **24.01.2020**

(51) International Patent Classification (IPC):
**A61G 5/10** *(2006.01)*        **A61G 7/057** *(2006.01)*
**A61H 23/02** *(2006.01)*

(52) Cooperative Patent Classification (CPC):
**A61G 5/1045; A61G 5/10; A61G 7/0573;**
**A61H 23/0263; G16H 40/63; G16H 40/67;**
A61G 5/1054; A61G 2203/30; A61G 2203/34;
A61G 2203/36; A61G 2203/40; A61G 2203/44;
A61H 2201/0134; A61H 2201/0149;
A61H 2201/1215;        (Cont.)

(86) International application number:
**PCT/AU2020/050044**

(87) International publication number:
**WO 2020/150783 (30.07.2020 Gazette 2020/31)**

(54) **SUPPORT CUSHION**

STÜTZKISSEN

COUSSIN DE SOUTIEN

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **25.01.2019  AU 2019900239**

(43) Date of publication of application:
**01.12.2021  Bulletin 2021/48**

(73) Proprietor: **RC Services Australia Pty Ltd**
**Parkinson QLD 4115 (AU)**

(72) Inventors:
• **CREALEY, Sean**
**Parkinson, Queensland 4115 (AU)**
• **REGAN, Justin**
**Parkinson, Queensland 4115 (AU)**
• **RICHARDSON, Kevin**
**Parkinson, Queensland 4115 (AU)**

(74) Representative: **Louis Pöhlau Lohrentz**
**Patentanwälte**
**Merianstrasse 26**
**90409 Nürnberg (DE)**

(56) References cited:
**AU-A1- 2013 201 721        CN-A- 107 361 930**
**CN-A- 109 512 597        CN-U- 204 015 808**
**DE-U1-202012 012 623        US-A- 6 030 351**
**US-A- 6 053 880        US-A1- 2013 180 530**
**US-B2- 8 038 632**

(52) Cooperative Patent Classification (CPC): (Cont.)
A61H 2201/1623; A61H 2201/1633;
A61H 2201/169; A61H 2201/5007;
A61H 2201/5023; A61H 2201/5035;
A61H 2201/5043; A61H 2201/5071;
A61H 2201/5084; A61H 2201/5097;
A61H 2203/0431; A61H 2205/081; A61H 2205/086;
A61H 2230/625; A61H 2230/805; G16H 20/30

**Description**

FIELD OF THE INVENTION

The present invention relates to the field of medical devices. In particular, the invention relates to a cushion for a chair or bed, More particularly, the invention relates to a cushion for a wheelchair that may be useful for prevention or treatment of pressure ulcers. Such a device is disclosed in the US20130180530.

BACKGROUND TO THE INVENTION

An immobile person may be at risk of developing pressure ulcers, also known as decubitus ulcers or bed sores. The problem is particularly pronounced in patients lying in a bed for an extended period of time but is also manifest in patients confined to a wheelchair. Although the following discussion is focussed on wheelchairs it will be appreciated that the same considerations may apply to any user who remains in one position for lengthy periods without the ability to move.

Persons confined to a wheelchair may have upper body mobility yet may not have the ability to change their position in the wheelchair. As a result, the blood circulation around the buttocks and upper thighs may be restricted. The precise reasons for pressure ulcers are not fully understood but there is a high correlation between poor blood flow, lack of movement, load from weight, moisture and sheer with the development of pressure ulcers and similar problems. Skin breakdown is thought to result from the impact of load and pressure on the skin which restricts the skin blood flow which is critical for skin tissue viability.

It is known to treat or prevent pressure ulcers and the like by massaging the areas of the body prone to the ulcers. One particular known technique and device is described in Great Britain patent application number GB2528967 assigned to Vibrant Medical Limited. This patent application describes a support structure for a patient at risk of ulcers and is particularly focussed on a vibrating massage. However, the description makes brief reference to the possibility of incorporating vibration into a cushion and states "[The] cushion may be incorporated into the seat of a wheelchair, or a similar chair in which a patient of limited mobility may be expected to spend extended periods of time. The cushion 40 incorporates a vibration unit comprising a motor 16 connected to a flexible plastics frame 12, to provide vibrations to an occupant sitting on surface 14a through first layer of foam 14'. Cushion 40 may further comprise control hardware in communication with vibration motor 16, so as to provide vibration therapy to treat or prevent pressure ulcers for the patient sitting on cushion 40."

The Vibrant Medical device is described as employing cycloidal vibrations but does so in a limited way which fails to achieve a useful level of beneficial effect. US6030351 also discloses a pressure relief reminder and compliance system to prevent the formation of pressure sores. In particular, US6030351 is directed to the measurement of "inter-relief intervals" to ensure that pressure relief occurs after a prolonged period of accumulated pressure.

SUMMARY OF THE INVENTION

The invention is disclosed by the claims. The invention resides in a support cushion to ameliorate the incidence of pressure ulcers, the support cushion comprising:

> an open or closed cell support structure;
> at least one cavity formed within the support structure;
> at least one vibrational device located within at least one of the cavities and imparting vibration to the support structure;
> a controller controlling operation of the vibrational device to adjust one or more of intensity, duration and location of vibrations in a predetermined manner
> according to any one of a number of selectable programmes;
> at least one sensor adapted to measure a weight distribution of a user on the support cushion; and
> a computer application programme;
> characterised in that the computer application programme is executable by a processor on a mobile computing device wirelessly connected to the controller to select the selected programme and to receive data from the controller, the mobile computing device comprising a graphical user interface.

Preferably the controller is a microprocessor device programmed to control the vibrational device, the vibrational device operating according to a selected programme.

Suitably the microprocessor device is connected to a control panel having an interface which allows selection of one of a number of selectable programmes.

The control panel is preferably incorporated into the support structure and/or wirelessly connected to the microprocessor device and/or incorporated into an available computing device programmed to control the microprocessor device.

The selected programme is preferably customisable to adjust the intensity and/or duration and/or location of vibrations.

The at least one sensor provides input to the controller, which may adjust the intensity and/or duration and/or location of vibrations based on the input from the sensor.

An indication may be provided if a prolonged disproportionate distribution of weight on the support cushion is sensed.

The computer application program may display a map of weight distribution as sensed by the at least one sensor on the graphical user interface.

Suitably, the computer application program records, and/or provides on the graphical user interface, a weight distribution history log of the support cushion as sensed by the at least one sensor.

The computer application may provide visual and/or audio reminders for movement of the user.

Preferably the at least one sensor is comprised of at least one set of sensing device pairs, each of the sensing device pairs comprising at least one proximity sensor and one accelerometer.

The sensor may be comprised of four sets of sensing device pairs, the four sets of sensing device pairs arranged in a quadrant layout.

Preferably the quadrant layout of the sensors monitor centre of gravity of the user to determine a symmetry of posture.

Preferably the symmetry of posture is determined by a set of ratios between the sensors arranged in the quadrant layout.

Suitably the controller turns off the at least one vibrational device when the at least one sensor determines that no user is present on the support cushion.

Preferably the support structure is comprised of one or more of; latex, polymer materials, multi-density foam, memory foam, anti-pressure gel.

Suitably the at least one cavity is sized and shaped to receive the at least one vibrational device. There may suitably be two or more cavities each receiving a vibrational device.

The vibrational device is preferably a cycloidal vibration device imparting cycloidal vibrational therapy to a user positioned upon the support structure. The cycloidal vibration device suitably incorporates a brushless servo motor.

The support cushion may be adapted for use as one of the following: seat cushion; wheelchair cushion; back-rest lumbar cushion; pet-bed cushion; mattress cushion.

Further features and advantages of the present invention will become apparent from the following detailed description.

## BRIEF DESCRIPTION OF THE DRAWINGS

To assist in understanding the invention and to enable a person skilled in the art to put the invention into practical effect, preferred embodiments of the invention will be described by way of example only with reference to the accompanying drawings, in which:

FIG 1 is a sketch of the invention in use on a wheelchair;

FIG 2 shows a first embodiment of a support cushion;

FIG 3 shows a second embodiment of a support cushion;

FIG 4 shows an exploded view of the embodiment of FIG 1;

FIG 5 shows a suitable cycloidal vibration therapy motor;

FIG 6 shows a third embodiment of a support cushion;

FIG 7 shows an embodiment of sensors for the support cushion;

FIG 8 shows an exploded view of a fourth embodiment of a support cushion;

FIG 9 is a flowchart of a programme for a control device; and

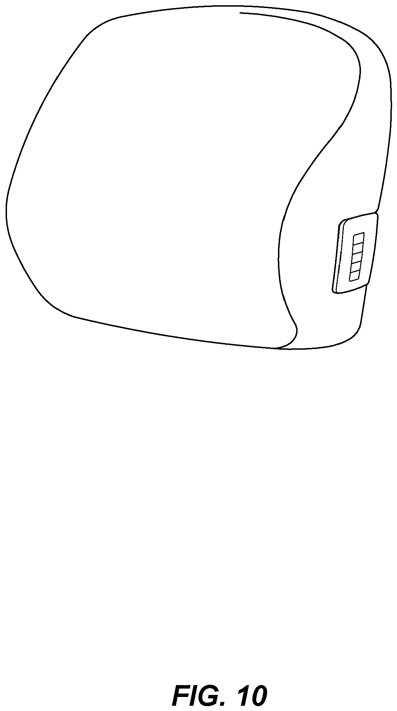

FIG 10 shows a fifth embodiment of a support cushion.

DETAILED DESCRIPTION OF THE INVENTION

Embodiments of the present invention reside primarily in a support cushion and a method of vibrating the support cushion. Accordingly, the integers of the cushion and the method steps have been illustrated in concise schematic form in the drawings, showing only those specific details that are necessary for understanding the embodiments of the present invention, but so as not to obscure the disclosure with excessive detail that will be readily apparent to those of ordinary skill in the art having the benefit of the present description.

In this specification, adjectives such as first and second, left and right, and the like may be used solely to distinguish one element or action from another element or action without necessarily requiring or implying any actual such relationship or order. Words such as "comprises" or "includes" are intended to define a non-exclusive inclusion, such that a process, method, article, or apparatus that comprises a list of elements does not include only those elements but may include other elements not expressly listed, including elements that are inherent to such a process, method, article, or apparatus.

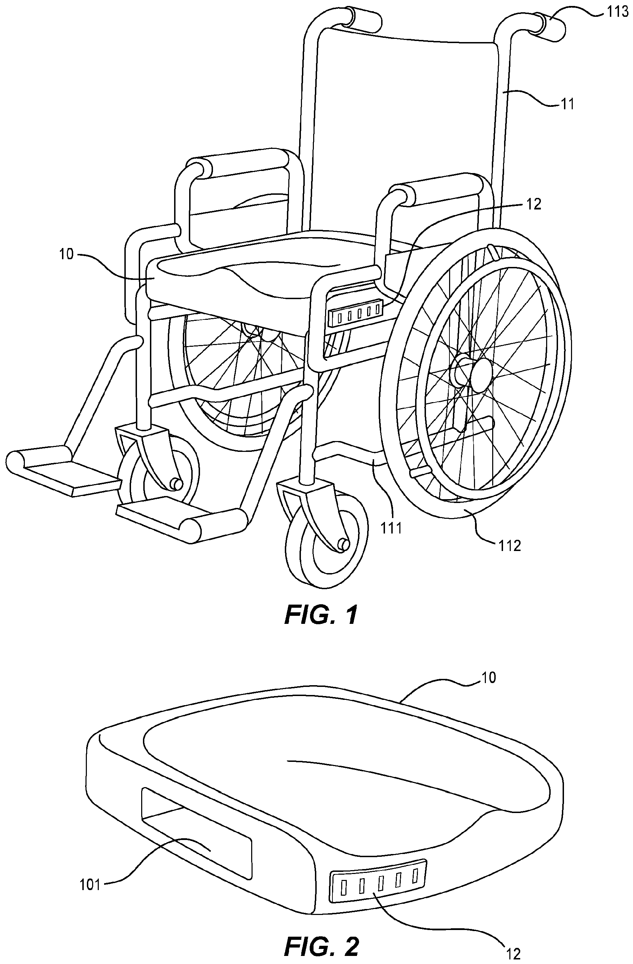

Referring to FIG 1 there is a shown a sketch of a cushion 10 on a typical wheelchair 11. The wheelchair 11 conventionally comprises a frame 111 with wheels 112 and push handles 113. The wheelchair 11 may be electric with a motor (not shown).

One embodiment of the cushion 10 is shown in FIG 2. The cushion 10 is of a size and shape to fit the wheelchair 11. The cushion 10 is made from closed-cell polyurethane or other suitable material. A cavity 101 is formed towards the middle of the cushion 10. The cavity 101 extends from the side or front of the cushion towards the opposite side or back. A plug (not shown) may be used to close the cavity once a cycloidal vibration motor is fitted in the cavity 101. A control panel 12 is moulded into the cushion at some position. In FIG 1 the control panel 12 is shown on the left side of the support cushion 10 and in FIG 2 the control panel 12 is shown on the left front of the support cushion 10. The specific location of the control panel 12 is not critical.

It will be noted that the top of the support cushion is moulded to provide comfort to the user. Although a particular design is depicted it is not essential to the invention that the support cushion have any specific size or shape. Indeed, the support cushion could be large and configured as a bed mattress.

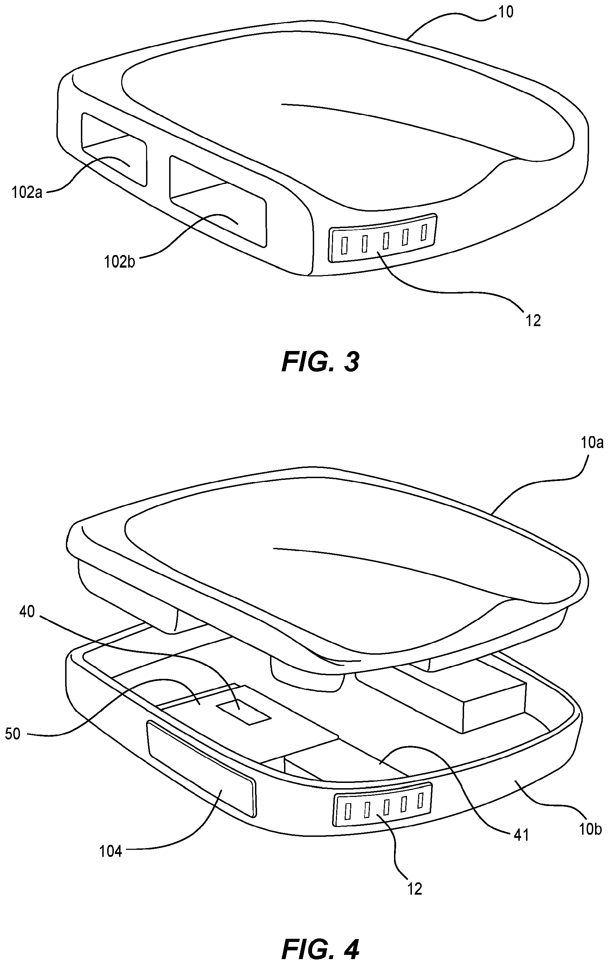

An alternate embodiment is shown in FIG 3. In the embodiment of FIG 3 the cushion 10 has two cavities, 102a, 102b. The cavities have a similar structure to the cavity of the embodiment of FIG 2. Two cavities allow for a greater variation in the programme of vibrations than is possible with the single cavity of FIG 2. In other embodiments (not shown) there are three or more cavities.

The support cushion 10 is conveniently moulded in two parts as shown in FIG 4. Fig 4 shows an upper part 10a which is designed for the comfort of the user positioned on the cushion. The lower part 10b is designed to contain the cycloidal vibration motor 50 and the controller 40, including the control panel 12, in electronics housing 41. A plug 104 is shown closing the cavity 101. The location of a cycloidal vibration motor 50 and electronics housing 41 are shown located in the lower part 10b.

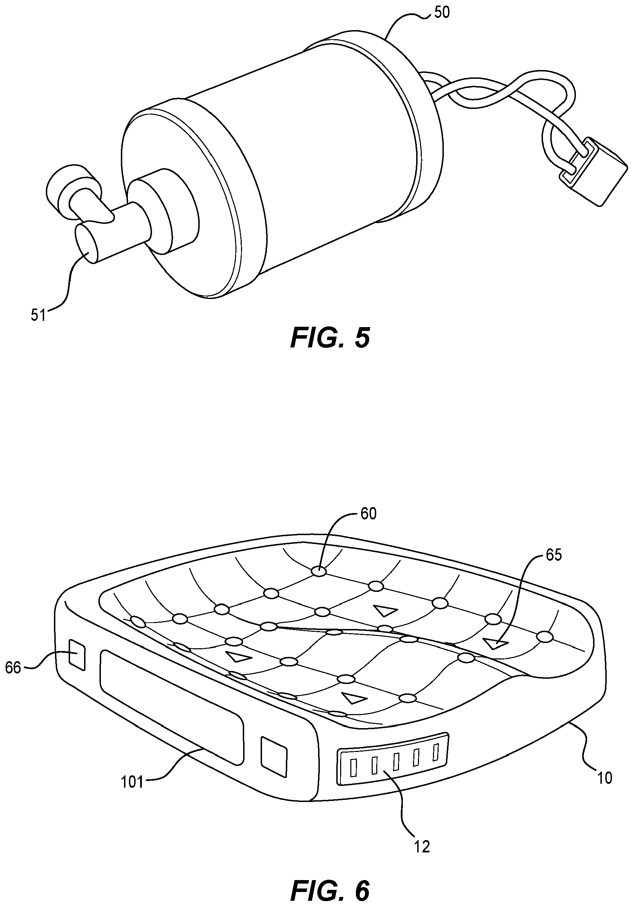

A suitable motor 50 for imparting cycloidal vibration therapy is shown in FIG 5. The motor is a compact DC brushless motor that can be battery powered. A suitable power source for the motor 50 or other electrical components of the support cushion 10 is a single cell, high capacity, rechargeable Lithium Ion battery which may be conveniently located in electronics housing 41. The battery is conveniently rechargeable via USB but may also be recharged from a mains adaptor. Battery condition may be indicated by a coloured LED mounted on a front surface of the cushion. Battery management is built into the controller.

The motor drives an eccentric weight 51 that imparts a vibration to the cushion 10. The frequency of vibration is determined by the speed of rotation of the eccentric weight which is in turn determined by the RPM of the motor 50. The motor may be servo locked to a crystal reference. A typical vibration frequency is 30-50 Hz but this may be adjustable in a range. The amplitude of vibration is typically 0.1 -0.8 mm.

Operation of the vibration device is via the controller 40 that may include a control panel 12 incorporated into the support cushion 10 or support structure. The controller 40 may be a microprocessor device programmed to control the vibrational device, the vibrational device operating according to the user selected programme. The microprocessor device or controller is connected to a control panel 12 having an interface which allows the selection one of the number of selectable programmes. The control panel 12 is shown most clearly in FIG 2 and FIG 3. The control panel 12 comprises push buttons on a control decal on the front outside of the cushion 10, with indicator lights to confirm operation.

In alternative embodiments, the control panel is incorporated into the support structure and/or wirelessly connected to the microprocessor device and/or incorporated into an available computing device programmed to control the microprocessor device. As an example, part of the controller may be incorporated with the motor 50 and be accessible via a remote link such as Bluetooth® or WiFi®. The controller is thereby wirelessly connected to a computer application program that is executable by a processor on a mobile computing device, the computing device comprising a graphical user interface. In this embodiment the controller is accessible via an application running on a smartphone, tablet or desktop computer. Various functionality options are described below which may not be available in every embodiment. An advantage of the remote link controller is that it avoids the need for cables between the cushion 10 and the control panel 12. On the other hand, a control panel has the advantage of push buttons for self-activation by the user positioned on the cushion 10.

The controller, whether via a remote link or local control panel, has functionality to start and stop vibration therapy as well as various indications to supplement the operation. As described below, the supplementary functionality may include a graphical display of pressure (weight) spread on the cushion, battery charge indication, and operating programme.

Embedded in the cushion 10 may be at least one sensor, embodied as pressure sensors 60, to measure the weight distribution of a patient sitting on the cushion 10. The pressure sensors 60 are able to monitor the centre of gravity of the user and determine posture symmetry via localised foam compression (including: left, right, front, and rear).

Exemplary locations of the pressure sensors 60 are shown in the embodiment of the cushion shown in FIG 6. In one embodiment the sensor is in the form of pressure sensors 60 arranged as an array distributed across the cushion. Signals from the pressure sensors 60 are analysed by the controller 40. In one embodiment a graphical display of pressure distribution is provided by the controller 40. The graphical display may be transmitted to a smartphone, tablet or desktop computer. In another embodiment a display is provided with the control panel 12. The pressure distribution is used to select an appropriate treatment regime as outlined below. The selection may be made by the user of the cushion, a clinician/carer or may be automatically selected from available programmes. By way of example, if the pressure distribution indicates more pressure on one buttock than the other the treatment regime may provide different frequency or duration of vibration to each buttock.

The at least one sensor, presently embodied as pressure sensors 60, can therefore be suitably used as inputs to the controller 40, adapted to adjust the intensity and/or duration and/or location of vibrations depending on the mapped weight distribution. Alternatively, the controller 40 can also work as a switch to turn off the at least one vibrational device when the at least one sensor determines that no user is present on the support cushion 10. This may pause an active session when a user leaves the cushion mid-cycle or prevent a scheduled therapeutic massage cycle from initiating when the user is absent from the cushion.

The pressure sensors 60 may be capacitive sensors, force sensitive resistors, piezoelectric devices, an array of strain gauges or stretchable conductive cloth.

The cushion 10 may also incorporate movement sensors such as accelerometers 66 to detect movement of the wheelchair and/or movement of the user positioned on the cushion. The data from the movement sensors 66 provide information to a carer that the user positioned on the cushion may require attention. This information provided to a carer may also take on the form of a visual and/or audio reminder. Another purpose of the accelerometer is to detect movement of the cushion. Tilt-in-space chairs are known (such as recliner chairs) that will tilt the entire cushion thereby causing a weight redistribution. The accelerometers will detect such movement. In another embodiment the range of movement considered to be significant may be set. For instance, a movement of less than a certain angle may be disregarded and a movement greater than a certain angle may generate a different indication. A very large movement may generate an indication of a risk that the user has fallen from the cushion 10.

The at least one sensor is also able to determine occupancy of the cushion. In a preferred embodiment, the sum of all pressure sensors 60 is calculated against a threshold to suit the characteristics of the user and/or cushion and/or chair.

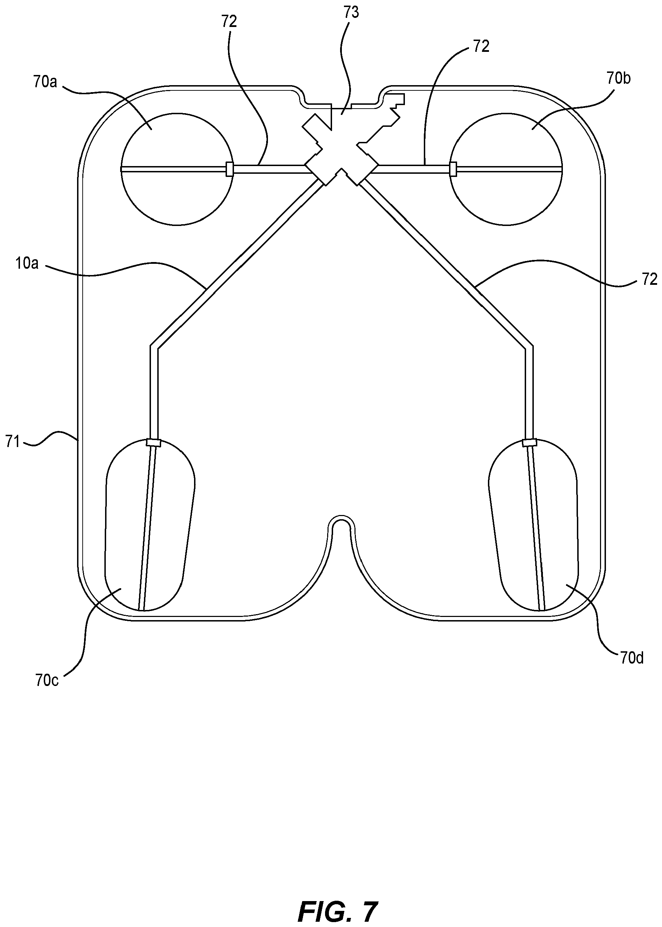

In a preferred embodiment for a seat cushion, four high precision capacitive proximity sensing elements (70a, 70b, 70c and 70d) are positioned on a plate 71 that is positioned below the upper part 10a of the support cushion 10. The sensing elements are aligned with the left buttock, right buttock, left thigh and right thigh as shown in FIG 7. The sensing elements may suitably be capacitive elements formed from a dielectric between two large parallel plates forming part of a tuned circuit having a resonant frequency excited by an active capacitance to digital converter device. Compression of adjacent material (foam) by the weight of a user causes a change in the capacitance of the system, which translates to a shift in resonant frequency, which is detected and proportional to the level of foam compression. Refinement of the shape and layout of the sensing elements allow sensing regions to have an effective sensing range equal to the thickness of the cushion, and shielded from the underside to prevent interaction with any cushion frames.

The signal from each sensing element is communicated to a control circuit 73 on signal lines 72. Further processing may be provided by control circuit 73. Control circuit 73 also provides an interface to the controller 40.

Each proximity sensing element can measure extremely small changes in capacitance due to small changes in proximity within the range of the cushion thickness. Both sensing regions and the cushion structure have bilateral symmetry. This allows ratiometric measurements to be made to determine the centre of gravity of the user, and thereby determine posture symmetry.

The left/right posture symmetry can be determined by the ratio between:

(Left thigh proximity + Left buttock proximity) : (Right thigh proximity + Right buttock proximity)

The front/back posture symmetry can be determined by the ratio between:

(Right buttock proximity + Left buttock proximity) : (Right thigh proximity + Left thigh proximity).

It should be noted that the sensor configuration of FIG 7 does not measure weight or pressure directly but measures posture symmetry (left-right-front-rear) and cushion occupancy.

As with the embodiment of FIG 6, each proximity sensing element may also include an accelerometer, suitably precision three axis accelerometers. The combination of proximity sensors and accelerometers allows the sensor to determine when the user has re-positioned. If specified relief periods are met, indications and notifications may be reset. Chairs fitted with active tilt in space (TIS) systems can also be monitored to determine that the chair cushion has moved from the horizontal to a specified position within a range of positions. Again, once specified relief periods are met, indications and notifications may be reset.

The ratiometric left/right measurements also reduce measurement errors from noise, offsets, thermal drift, or foam degradation, as these changes will largely be common to the cushion body as a whole.

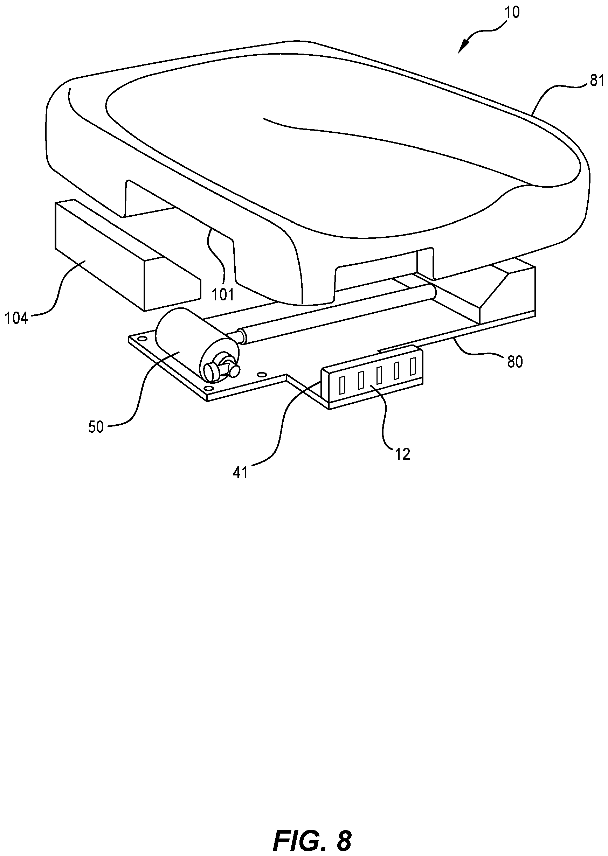

Another embodiment of the support cushion 10 is shown in FIG 8. In this embodiment the motor 50, electronics housing 41 and control panel 12 are mounted on a base plate 80. An upper part 81 of the support cushion 10 is attached to the base plate to assemble the support cushion 10. The plug 104 closes the cavity 101 as previously described. It is envisaged that the embodiment of FIG 8 may have advantage in imparting vibration to the cushion 10. Furthermore, the base plate 80 provides a useful platform for location of additional sensors.

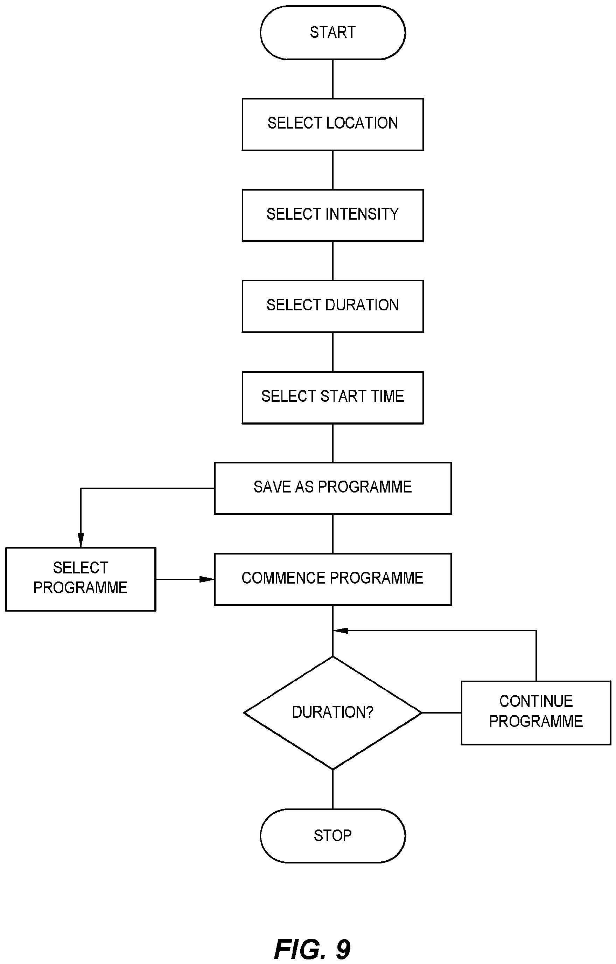

Referring to FIG 9 there is shown a flowchart of the process of programming and operating the controller to control the vibrations of the cycloidal vibration device. The first part of the flowchart of FIG 9 describes setting up the programmes. This may be done as a factory pre-set or can be performed by a user and/or carer. The selected programme is customisable for adjusting the intensity and/or duration and/or location of vibrations. The first step of commencing the set-up process is to select the location of the vibrations. If there is only one cycloidal vibration device this step may be redundant but if there are two or more cycloidal vibration devices the following steps may be performed for each cycloidal vibration device so that the vibration programme is different at different locations and/or different times.

After the location is selected the intensity of vibration is selected, intensity may also be pre-set. The intensity may be selected on a suitable scale, such as a ten-point scale, ranging from minimum intensity to maximum intensity. The intensity of vibration is determined by the frequency. The frequency may be varied in the range from around 30 Hz to about 50Hz. The duration of the vibration therapy is selected and this will typically be for a period of time from 15 minutes to 50 mins. The duration may also be repeated, for example 40 minutes of vibration followed by a rest of one to two hours followed by another 40 minutes of vibration that may also be repeated a third time.

Finally, a start time is selected. This may be an immediate start, a programmed start after a fixed delay or a start at a particular time or times, for example at 9am every day. To facilitate this function the controller may incorporate a real-time clock and calendar integrated circuit. Such devices are readily available. The device may also provide event logging capacity as discussed below.

In one embodiment the controller includes pre-set programmes in which location, frequency/speed of vibration, time and duration are activated at the push of one button on the control panel and/or the smartphone application, suitably at the start of the day.

The designed programme may be saved and suitably numbered (programme 1, programme 2, etc). The save step is optional, so for user selected parameters the programme may simply run as a one-off application.

For automated operation a programme may be selected from the stored programmes and commenced. The stored programmes may be pre-set and supplied to the user, or programmes specifically designed by or for the user.

The programme periodically checks the duration via a time loop and continues until the full duration elapses.

The inventors have found that the programmable vibrations have particular advantage compared to the continuous operation of the prior art. By way of example, the following programmes are considered to be beneficial and are expected to provide particular advantage:

- Three 30 min treatments per day at a frequency of 30-40hz timed at 1.5-2 hours apart. This may be operated from the one push button on the control panel that will immediately start the first 30-40 mins treatment. The operator may be able to change or adjust frequency/speed of vibration of the motor and/or the duration;

- The frequency slowly increases from 30Hz to 50Hz over a 90 second period, which is not noticeable by the patient. It is expected that applying a range of vibrational frequencies over a 30 minute treatment will improve blood flow;

- Four 15 minute treatments spread over an 8 hour timed programme. This may provide more regular improvement of blood flow in risk locations;

- Treatment frequency and duration selected according to weight. A person of average weight may have a 30 minute treatment at 30Hz whereas a heavier person may have a 35 minute treatment at 40Hz. A larger person may have a 40 minute treatment at 50Hz.

The programme outlined above is provided by way of example only. Other programmes may be appropriate for certain patients in certain situations and may have particular, innovative advantage. The following variations are available:

Location: Right buttock; right thigh; left buttock; left thigh; any combination of right, left, buttock and thigh;

Frequency: 30Hz to 50Hz

Intensity: 0.1mm to 0.8mm amplitude of movement

Duration: 30 minutes to 3 hours in 15 minute increments, repeatable with pause between treatments of 30 minute to 6 hours in 30 minute increments;

The controller and/or computer application program records, and/or provides on the graphical user interface, a pressure distribution history log of the support cushion 10 as sensed by the at least one sensor. Long term posture trends can also be logged according to the symmetry of user loading as determined by the at least one sensor. The controller and/or computer application program also maintains various logs of activity. The logs may include, for example, massage time per session per day, chair movements, and patient movements. The logs are analysed for clinical assessment leading to variation of the programmes for improved efficacy. The software will log data of pressure mapping and sensing. The computer application program can also provide on the graphical user interface the weight (pressure) distribution map of the support cushion 10 as sensed by the at least one sensor. Optionally, a graphical representation of the user's posture symmetry may also be provided. Visual indication of a user's position of symmetry between Left/Right and Front/Back may also be displayed, for example, as a 2x2 grid arrangement with the following colours indicating positions:

Green spot - equilibrium (<+/- 5%).

Amber - moderately out of position (> 5% < 15%).

Red - extreme position, immediate reposition required (> 15%).

The data and history log may be automatically compiled and users and/or carers/nursing staff receive daily, weekly or monthly summary reports of usage, i.e. if the support cushion is activated each day, and also reports of posture trends. The log may provide a percentage breakdown indicating how much time a user has spent in balanced or imbalanced postures. Users and/or carers/nursing staff may then evaluate the data and, using standard support structures, make corrective changes to a user's posture.

The controller will also provide indications to users and or carer/nursing staff via their smartphone/tablet or device. The indication may be visual, audible, tactile or a combination such as a vibration and ring tone. The indication may be, for example, that the pressure sensor timer has expired and the patient needs to be repositioned to relieve the pressure. Alternatively, the indication may be that a prolonged disproportionate distribution of pressure on the support cushion 10 is sensed. A disproportionate distribution of pressure on the support cushion 10 may also indicate asymmetrical loading, and that the user's poster requires correction. The user may have slumped left, right, or forward. The indication is only reset once the pressure sensors have registered a release in pressure or return to symmetrical loading/posture by repositioning for a specified continuous period of time. The appropriate period of time may be adjusted by a health care professional to suit the needs of an individual user.

The controller may also log the time it takes from the activation of the pressure indication to repositioning and reset. The response data forms part of optional summary reporting that shows how quickly carer or nursing staff are attending to patients and can be a useful oversight and management tool. The family of users of the support cushion may also be able to have the summary reports sent to them for oversight. The data is also useful to home care services providing multiple and varied carers to one individual in their home to monitor adherence of staff to mandated usage of the support cushion.

The controller may also incorporate a network facility so that multiple support cushions may be monitored from a central location. This may be particularly useful in nursing home facilities with multiple cushion products in use. A central monitor may display all operating units in the nursing home facility so that operation can be checked. Reports can provide information on operational matters such as usage, response times and effectiveness.

The control panel may also include a flashing indicator light to give a local visual indication that it is time to reposition the user of the support cushion to relieve pressure. The optimum time to reposition a patient has been said to be every 15-20 minutes.

The controller may also be programmed to give a user of the support cushion an indication that they have been in the same position for an extended period of time and should reposition. The indication that it is time to move can be a short intense vibration provided by the cycloidal vibration motor.

Numerous other features may be incorporated into the controller to enhance the effectiveness of the support cushion. For instance, a vibration programme could be suspended or stopped if pressure is released from the cushion, such as by the user no longer sitting in the chair. Various parameters can be logged over time and used for diagnosis. Parameters may include pressure and location, movement, lack of movement, etc.

As mentioned, although particular designs are depicted it is not essential to the invention that the support cushion have any specific size or shape. The support cushion 10 may be moulded to provide postural support and/or adapted for use as a seat cushion; wheel chair cushion; back-rest lumbar cushion; pet-bed cushion; or mattress cushion. Fig 9 shows one such embodiment of the support cushion as a cushion for back-rest lumbar support.

The above description of various embodiments of the present invention is provided for purposes of description to one of ordinary skill in the related art.

**Claims**

1. A support cushion (10) to ameliorate the incidence of pressure ulcers, the support cushion (10) comprising:

   an open or closed cell support structure;
   at least one cavity (101) formed within the support structure;
   at least one vibrational device located within at least one of the cavities (101) and imparting vibration to the support structure;
   a controller (40) controlling operation of the vibrational device to adjust one or more of intensity, duration and location of vibrations in a predetermined manner according to a selected programme of any one of a number of selectable programmes;
   at least one sensor adapted to measure a weight distribution of a user on the support cushion (10); and
   a computer application programme;
   **characterised in that** the computer application programme is executable by a processor on a mobile computing device wirelessly connected to the controller (40) to select the selected programme and to receive data from the controller (40), the mobile computing device comprising a graphical user interface.

2. The support cushion (10) of claim 1, wherein the controller (40) is a microprocessor device programmed to control the vibrational device, the vibrational device operating according to the selected programme.

3. The support cushion (10) of claim 2, wherein the microprocessor device is connected to a control panel having an interface which allows selection of the selected programme.

4. The support cushion (10) of claim 3, wherein the control panel is incorporated into the support structure and/or wirelessly connected to the microprocessor device and/or incorporated into an available computing device programmed to control the microprocessor device.

5. The support cushion (10) of claim 1, wherein the selected programme is customisable to adjust the intensity and/or duration and/or location of vibrations.

6. The support cushion (10) of claim 1, wherein the at least one sensor is suitably used as inputs to the controller (40),

adapted to adjust the intensity and/or duration and/or location of vibrations.

7.  The support cushion (10) of claim 1, wherein an indication is provided if a prolonged disproportionate distribution of weight on the support cushion (10) is sensed.

8.  The support cushion (10) of claim 7, wherein the computer application provides visual and/or audio reminders for movement of the user.

9.  The support cushion (10) of claim 1, wherein the computer application program provides on the graphical user interface a display of a map of the weight distribution of the support cushion (10) as sensed by the at least one sensor.

10. The support cushion (10) of claim 1, wherein the computer application program records, and/or provides on the graphical user interface, a pressure distribution history log of the support cushion (10) as sensed by the at least one sensor.

11. The support cushion (10) of claim 1, wherein the at least one sensor is comprised of at least one set of sensing device pairs, each of the sensing device pairs comprising at least one proximity sensor and one accelerometer.

12. The support cushion (10) of claim 1, wherein the sensor is comprised of four sets of sensing devices, the four sets of sensing devices arranged in a quadrant layout.

13. The support cushion (10) of claim 1, wherein the controller (40) turns off the at least one vibrational device when the at least one sensor determines that no user is present on the support cushion (10).

14. The support cushion (10) of claim 1, wherein the at least one cavity (101) is sized and shaped to receive at least one vibrational device and wherein the at least one vibrational device is a cycloidal vibration device imparting cycloidal vibrational therapy to a user positioned upon the support cushion (10).


**Patentansprüche**

1.  Stützkissen (10) zur Verringerung des Auftretens von Druckgeschwüren, wobei das Stützkissen (10) umfasst:

    eine offene oder geschlossene Zellstützstruktur;
    mindestens einen Hohlraum (101), der in der Stützstruktur ausgebildet ist;
    mindestens eine Vibrationsvorrichtung, die sich in mindestens einem der Hohlräume (101) befindet und die Stützstruktur in Vibration versetzt;
    ein Steuergerät (40), das den Betrieb der Vibrationsvorrichtung steuert, um die Intensität und/oder die Dauer und/oder den Ort der Vibrationen in einer vorbestimmten Weise gemäß einem ausgewählten Programm aus einer beliebigen Anzahl von auswählbaren Programmen einzustellen;
    mindestens einen Sensor, der geeignet ist, die Gewichtsverteilung eines Benutzers auf dem Stützkissen (10) zu messen; und
    ein Computer-Anwendungsprogramm;
    **dadurch gekennzeichnet, dass** das Computer-Anwendungsprogramm von einem Prozessor auf einem mobilen Computergerät, das drahtlos mit der Steuerung (40) verbunden ist, ausführbar ist, um das ausgewählte Programm auszuwählen und Daten von der Steuerung (40) zu empfangen, wobei das mobile Computergerät eine grafische Benutzeroberfläche umfasst.

2.  Stützkissen (10) nach Anspruch 1, wobei die Steuerung (40) eine Mikroprozessorvorrichtung ist, die so programmiert ist, dass sie die Vibrationsvorrichtung steuert, wobei die Vibrationsvorrichtung gemäß dem ausgewählten Programm arbeitet.

3.  Stützkissen (10) nach Anspruch 2, wobei die Mikroprozessorvorrichtung mit einem Bedienfeld verbunden ist, das eine Schnittstelle aufweist, die die Auswahl des gewählten Programms ermöglicht.

4.  Stützkissen (10) nach Anspruch 3, wobei das Bedienfeld in die Stützstruktur integriert ist und/oder drahtlos mit der Mikroprozessorvorrichtung verbunden ist und/oder in ein verfügbares Computergerät integriert ist, das zur Steuerung der Mikroprozessorvorrichtung programmiert ist.

**5.** Stützkissen (10) nach Anspruch 1, wobei das gewählte Programm anpassbar ist, um die Intensität und/oder die Dauer und/oder den Ort der Vibrationen einzustellen.

**6.** Stützkissen (10) nach Anspruch 1, wobei der mindestens eine Sensor in geeigneter Weise als Eingang für die Steuerung (40) verwendet wird, um die Intensität und/oder die Dauer und/oder den Ort der Vibrationen einzustellen.

**7.** Stützkissen (10) nach Anspruch 1, wobei ein Hinweis gegeben wird, wenn eine anhaltende unverhältnismäßige Gewichtsverteilung auf dem Stützkissen (10) festgestellt wird.

**8.** Stützkissen (10) nach Anspruch 7, wobei die Computeranwendung visuelle und/oder akustische Erinnerungen für die Bewegung des Benutzers bereitstellt.

**9.** Stützkissen (10) nach Anspruch 1, wobei das Computer-Anwendungsprogramm auf der grafischen Benutzeroberfläche eine Anzeige einer Karte der Gewichtsverteilung des Stützkissens (10) bereitstellt, wie sie von dem mindestens einen Sensor erfasst wird.

**10.** Stützkissen (10) nach Anspruch 1, wobei das Computer-Anwendungsprogramm ein Protokoll der Druckverteilung des Stützkissens (10), wie sie von dem mindestens einen Sensor erfasst wird, aufzeichnet, und/oder auf der grafischen Benutzeroberfläche bereitstellt.

**11.** Stützkissen (10) nach Anspruch 1, wobei der mindestens eine Sensor aus mindestens einem Satz von Sensorvorrichtungspaaren besteht, wobei jedes der Sensorvorrichtungspaare mindestens einen Näherungssensor und einen Beschleunigungssensor umfasst.

**12.** Stützkissen (10) nach Anspruch 1, wobei der Sensor aus vier Sätzen von Sensorvorrichtungen aufgebaut ist, wobei die vier Sätze von Sensorvorrichtungen in einer Quadrantenanordnung angeordnet sind.

**13.** Stützkissen (10) nach Anspruch 1, wobei die Steuerung (40) die mindestens eine Vibrationsvorrichtung ausschaltet, wenn der mindestens eine Sensor feststellt, dass sich kein Benutzer auf dem Stützkissen (10) befindet.

**14.** Stützkissen (10) nach Anspruch 1, wobei der mindestens eine Hohlraum (101) so bemessen und geformt ist, um mindestens eine Vibrationsvorrichtung aufzunehmen, und wobei die mindestens eine Vibrationsvorrichtung eine zykloidische Vibrationsvorrichtung ist, die einem auf dem Stützkissen (10) positionierten Benutzer eine zykloidische Vibrationstherapie vermittelt.

**Revendications**

**1.** Un coussin de soutien (10) pour atténuer l'incidence d'escarres, le coussin de soutien (10) comprenant :

une structure de soutien à cellules ouvertes ou fermées ;
au moins une cavité (101) formée à l'intérieur de la structure de soutien ;
au moins un dispositif vibratoire situé à l'intérieur d'au moins l'une des cavités (101) et transmettant une vibration à la structure de soutien ;
un dispositif de commande (40) commandant le fonctionnement du dispositif vibratoire pour régler un ou plusieurs de l'intensité, de la durée et de l'emplacement de vibrations d'une manière prédéterminée selon un programme sélectionné de l'un d'un nombre de programmes sélectionnables ;
au moins un capteur adapté pour mesurer une distribution de poids d'un utilisateur sur le coussin de soutien (10) ; et
un programme d'application informatique ;
**caractérisé en ce que** le programme d'application informatique est exécutable par un processeur sur un dispositif informatique mobile connecté sans fil au dispositif de commande (40) pour sélectionner le programme sélectionné et pour recevoir des données provenant du dispositif de commande (40), le dispositif informatique mobile comprenant une interface utilisateur graphique.

**2.** Le coussin de soutien (10) selon la revendication 1, dans lequel le dispositif de commande (40) est un dispositif à microprocesseur programmé pour commander le dispositif vibratoire, le dispositif vibratoire fonctionnant selon le programme sélectionné.

3. Le coussin de soutien (10) selon la revendication 2, dans lequel le dispositif à microprocesseur est connecté à un panneau de commande ayant une interface qui permet la sélection du programme sélectionné.

4. Le coussin de soutien (10) selon la revendication 3, dans lequel le panneau de commande est incorporé dans la structure de soutien et/ou connecté sans fil au dispositif à microprocesseur et/ou incorporé dans un dispositif informatique disponible programmé pour commander le dispositif à microprocesseur.

5. Le coussin de soutien (10) selon la revendication 1, dans lequel le programme sélectionné est personnalisable pour régler l'intensité et/ou la durée et/ou l'emplacement des vibrations.

6. Le coussin de soutien (10) selon la revendication 1, dans lequel l'au moins un capteur est utilisé de manière appropriée comme entrées dans le dispositif de commande (40), adaptées pour régler l'intensité et/ou la durée et/ou l'emplacement de vibrations.

7. Le coussin de soutien (10) selon la revendication 1, dans lequel une indication est fournie si une distribution de poids disproportionnée prolongée sur le coussin de soutien (10) est détectée.

8. Le coussin de soutien (10) selon la revendication 7, dans lequel l'application informatique fournit des rappels visuels et/ou audio pour le mouvement de l'utilisateur.

9. Le coussin de soutien (10) selon la revendication 1, dans lequel le programme d'application informatique fournit sur l'interface utilisateur graphique un affichage d'une carte de la distribution de poids du coussin de soutien (10) telle que détectée par l'au moins un capteur.

10. Le coussin de soutien (10) selon la revendication 1, dans lequel le programme d'application informatique enregistre et/ou fournit sur l'interface utilisateur graphique, un fichier historique de distribution de pression du coussin de soutien (10) telle que détectée par l'au moins un capteur.

11. Le coussin de soutien (10) selon la revendication 1, dans lequel l'au moins un capteur est composé d'au moins un ensemble de paires de dispositifs de détection, chacune des paires de dispositifs de détection comprenant au moins un capteur de proximité et un accéléromètre.

12. Le coussin de soutien (10) selon la revendication 1, dans lequel le capteur est composé de quatre ensembles de dispositifs de détection, les quatre ensembles de dispositifs de détection étant agencés selon une disposition en quadrant.

13. Le coussin de soutien (10) selon la revendication 1, dans lequel le dispositif de commande (40) désactive l'au moins un dispositif vibratoire lorsque l'au moins un capteur détermine qu'aucun utilisateur n'est présent sur le coussin de soutien (10).

14. Le coussin de soutien (10) selon la revendication 1, dans lequel l'au moins une cavité (101) est dimensionnée et formée pour recevoir au moins un dispositif vibratoire et dans lequel l'au moins un dispositif vibratoire est un dispositif vibratoire cycloïdal transmettant une thérapie vibratoire cycloïdale à un utilisateur positionné sur le coussin de soutien (10).

**FIG. 1**

**FIG. 2**

10

102a

102b

12

**FIG. 3**

10a

40

50

104

41

10b

12

**FIG. 4**

**FIG. 5**

**FIG. 6**

**FIG. 7**

**FIG. 8**

```
                    ┌─────────────┐
                    │    START    │
                    └──────┬──────┘
                           │
                 ┌─────────┴─────────┐
                 │  SELECT LOCATION  │
                 └─────────┬─────────┘
                           │
                 ┌─────────┴─────────┐
                 │  SELECT INTENSITY │
                 └─────────┬─────────┘
                           │
                 ┌─────────┴─────────┐
                 │  SELECT DURATION  │
                 └─────────┬─────────┘
                           │
                 ┌─────────┴─────────┐
                 │ SELECT START TIME │
                 └─────────┬─────────┘
                           │
                 ┌─────────┴─────────┐
          ┌──────│ SAVE AS PROGRAMME │
          │      └─────────┬─────────┘
          │                │
  ┌───────┴───────┐  ┌─────┴───────────────┐
  │    SELECT     │  │                     │
  │   PROGRAMME   │─►│ COMMENCE PROGRAMME  │
  └───────────────┘  └─────────┬───────────┘
                               │
                          ◄────────────────┐
                        ◇                  │
                       ╱ ╲        ┌────────┴────────┐
                      ◇DURATION?◇►│    CONTINUE     │
                       ╲ ╱        │   PROGRAMME     │
                        ◇         └─────────────────┘
                        │
                 ┌──────┴──────┐
                 │    STOP     │
                 └─────────────┘
```

*FIG. 9*

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130180530 A
- GB 2528967 A
- US 6030351 A